# EUROPEAN PATENT APPLICATION

(11) **EP 2 149 389 A2**
(43) Date of publication of application: **03.02.2010**
(21) Application number: 09009210.7
(22) Date of filing: 13.07.2009
(51) Int. Cl.: A61M 39/22

(54) **Medical stopcock with a cap**

(30) Priority: 28.07.2008 JP 2008193836
(71) Applicant: Tyco Healthcare Group LP, Mansfield, MA 02048 (US)
(72) Inventor: Kitani, Ichiro, Fukuroi Shizuoka 437-0004 (JP)
(74) Representative: Olsson, Carl H.S.

(57) **Abstract**

A medical stopcock (A) with a cap (18) comprises a chamber part (11); an upstream branch pipe (12), a downstream branch pipe (13) and a supplementary upstream branch pipe (14) which are connected to the chamber part, respectively; a rotary valve (15) which can link in communication and block the areas between the chamber part and the upstream branch pipe, downstream branch pipe and supplementary upstream branch pipe, respectively; and a cap which can be attached to and detached from the supplementary upstream branch pipe. Furthermore, the cap is provided with an insertion part (18a) to which a filter (19) is fitted, and the movement of liquid between the chamber part and the supplementary upstream branch pipe can be prevented when the cap is fitted to the supplementary upstream branch pipe with the insertion part inserted into the supplementary upstream branch pipe.

## Description

### [Technical Field]

The present invention relates to a medical stopcock with a cap, comprising a plurality of branch pipes which are linked to a plurality of transfusion tubes or the like for medical use, said stopcock enabling the communicating or blocked state of the branch pipes to be switched, and also enabling branch pipes which are not being used to be closed off.

### [Prior Art]

Certain liquids such as physiological saline and drug solutions etc. are conventionally supplied to a patient's body by linking a plurality of transfusion tubes to a medical stopcock provided with a plurality of branch pipes. In such cases there are medical stopcocks with a cap in which air is allowed to enter a branch pipe through which liquid is not allowed to pass (which is not being used) from among the plurality of branch pipes, and a cap is fitted to that branch pipe in order to prevent bacterial growth and soiling (see Patent Document 1, for example).

This medical stopcock with a cap (three-way stopcock with a cap) consists of a cylindrical body, first to third liquid ports which are formed at the outer peripheral surface of this body and link in communication with the space inside the body, and a cock which is rotatably attached to the body. The cap is then detachably fitted to one of the liquid ports, and the cock is provided with a receiving hole for fitting of the cap when it is not being used.

Patent Document 1] Japanese Unexamined Utility Model Application Publication H6-44554

### [Disclosure of the Invention]

However, with the medical stopcock with a cap described above, air is likely to accumulate inside the liquid port to which the cap is fitted when the cap is fitted to one of the liquid ports, and liquid flows from one of the remaining liquid ports to the other liquid port via the body. If this air is left as it is, air mixes in with the liquid flowing in the liquid flow channels. Furthermore, if the cap is removed from the liquid port and another line or syringe or similar is repeatedly attached to or detached from that liquid port with the air still remaining in that liquid port, bacteria may enter the liquid port, and bacterial growth is likely to occur because of the accumulation of liquid which causes air pockets. Consequently, it is necessary to expel the air inside the liquid port before the cap is fitted, but the operation to expel the air is troublesome.

The present invention has been devised in view of this situation, and it aims to provide a medical stopcock with a cap with which it is possible to link in communication or block any branch pipe from among a plurality of branch pipes, and also with which it is possible to easily expel air inside a branch pipe which is not being used.

In order to achieve the above aim, structural features of the medical stopcock with a cap according to the present invention lie in the fact that it is a medical stopcock with a cap, comprising: a chamber part; a main upstream branch pipe, downstream branch pipe and supplementary upstream branch pipe which are respectively connected to the chamber part; a rotary valve provided inside the chamber part which can form a liquid flow channel allowing communication between the chamber part and the main upstream branch pipe, the downstream branch pipe and the supplementary upstream branch pipe, respectively, and also which can block the area between the chamber part and the main upstream branch pipe, the area between the chamber part and the downstream branch pipe, and the area between the chamber part and the supplementary upstream branch pipe, respectively, by rotation of said valve in a certain direction; and a cap which can be attached to or detached from the supplementary upstream branch pipe, said medical stopcock with a cap being **characterized in that** the cap comprises a cylindrical part which can be inserted into the supplementary upstream branch pipe, and a filter attached to the cylindrical part allowing the passage of gas but not allowing the passage of liquid; and when the cap is attached to the supplementary upstream branch pipe with the cylindrical part inserted into the supplementary upstream branch pipe, liquid can no longer move between the chamber part and the supplementary upstream branch pipe.

The medical stopcock with a cap according to the present invention which is configured in the manner described above allows communication or blocking between the main upstream branch pipe, downstream branch pipe and supplementary upstream branch pipe, respectively, and the chamber part, by rotation of the rotary valve. Then, when liquid flows from the main upstream branch pipe towards the downstream branch pipe only and the supplementary upstream branch pipe is not being used, the cap with a filter is fitted to the supplementary upstream branch pipe. This filter allows the passage of gas only, liquid being unable to pass through it, and therefore even if air accumulates inside the supplementary upstream branch pipe when liquid flows from the main upstream branch pipe to the downstream branch pipe, this air can pass through the filter and escape outside.

In this case, the air is pushed out by the liquid pressure when the rotary valve is rotated to a position which allows the main upstream branch pipe, downstream branch pipe and supplementary upstream branch pipe to link in communication, respectively, via the chamber part so that liquid flows from the main upstream branch pipe towards the chamber part. By means of this, it is possible to prevent air from being delivered into the body when a drug solution or the like is supplied to a patient's body. Furthermore, the cap is fitted to the supplementary upstream branch pipe, and therefore it is possible to prevent bacteria and soiling from entering the liquid flow channel of the medical stopcock with a cap. In this way, if the air inside the liquid flow channel is first of all expelled, air does not subsequently permeate into the liquid flow channel, even if the rotary valve is rotated. Moreover, the specific direction in which the rotary valve is rotated is the axial direction of the rotary valve in a state in which the peripheral surface of the rotary valve is oriented towards the main upstream branch pipe, downstream branch pipe and supplementary upstream branch pipe.

Further structural features of the medical stopcock with a cap according to the present invention lie in the fact that the filter consists of a thin-film hydrophobic filter. In this case, the filter thickness is preferably of the order of 0.1 - 0.5 mm. This means that it is possible to reliably prevent liquid from passing through the filter. Furthermore, it is simple to fit the filter to the cylindrical part, and also the filter does not become readily detached from the cylindrical part.

Further structural features of the medical stopcock with a cap according to the present invention lie in the fact that a male thread is provided on the outer peripheral surface of the supplementary upstream branch pipe, an outer peripheral wall for covering the outer peripheral surface of the supplementary upstream branch pipe is provided on the cap, and also a female thread which can screw together with the male thread is provided on the inner peripheral surface of the outer peripheral wall. This means that the cap can be easily attached to or detached from the supplementary upstream branch pipe.

Further structural features of the medical stopcock with a cap according to the present invention lie in the fact that the filter is attached to the tip end opening of the cylindrical part, and the filter is positioned at the boundary area between the chamber part and the main upstream branch pipe. This means that the filter can be easily fitted to the cylindrical part. Furthermore, the inside of the supplementary upstream branch pipe does not constitute a liquid flow channel, and therefore there is no longer any space inside the supplementary upstream branch pipe where air accumulates.

Further structural features of the medical stopcock with a cap according to the present invention lie in the fact that a seal for sealing the area between the cylindrical part and the inner peripheral surface of the supplementary upstream branch pipe is provided around the circumference of the cylindrical part. This means that it is possible to reliably prevent air from entering the liquid flow channel of the medical stopcock with a cap from between the cylindrical part and the inner peripheral surface of the supplementary upstream branch pipe. In this case, a one-way valve which allows air to pass from the inside to the outside of the supplementary upstream branch pipe but which does not allow air to pass from the outside to the inside, is preferably provided adjacent to the filter. This means that it is possible to prevent air from entering the supplementary upstream branch pipe when the cap is removed from the supplementary upstream branch pipe to connect a transfusion tube to the supplementary upstream branch pipe in order to supply another liquid, once the air inside the supplementary upstream branch pipe has been expelled.

That is to say, when the cap is withdrawn from the supplementary upstream branch pipe, the area between the cylindrical part and the inner peripheral surface of the supplementary upstream branch pipe is sealed by means of the seal, and therefore there is negative pressure inside the supplementary upstream branch pipe, and the liquid surface rises as the cap is lifted up. Consequently, if the rotary valve is operated to block the area between the supplementary upstream branch pipe and the chamber part, with the cap having been withdrawn and the liquid surface having reached the opening of the supplementary upstream branch pipe, the liquid surface stays as it is. In this state, a transfusion tube can be connected to the supplementary upstream branch pipe without air entering the supplementary upstream branch pipe, by connecting the transfusion tube which is filled with another liquid to the supplementary upstream branch pipe.

### [Optimum Mode of Embodiment of the Invention]

### Mode of Embodiment 1)

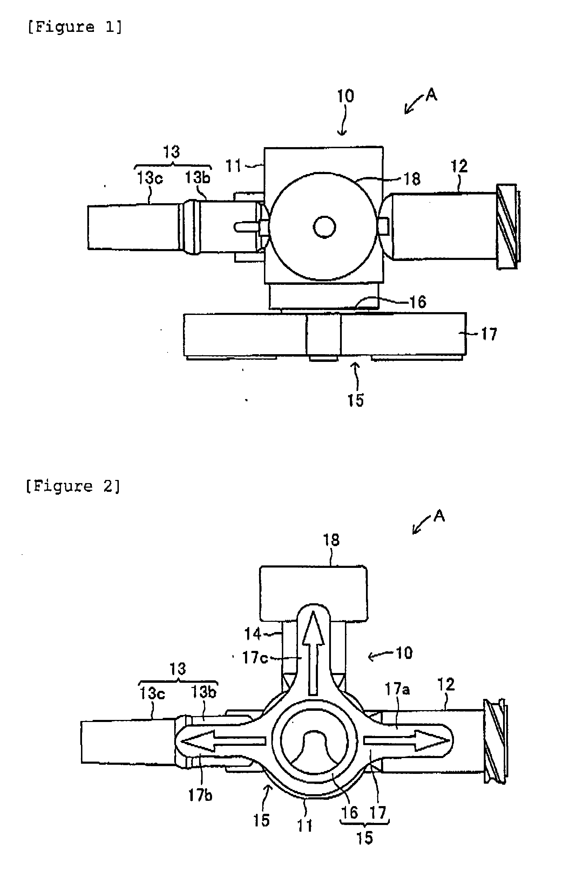

The medical stopcock with a cap according to Mode of Embodiment 1 of the present invention will be described below in detail. Figures 1 to 3 show a medical stopcock A with a cap, this medical stopcock A with a cap consisting of a medical stopcock main body 10, a rotary valve 15 rotatably fitted to the medical stopcock main body 10, and a cap 18. The medical stopcock main body 10 consists of a chamber part 11 formed as a cylindrical shape which is short in the axial direction, and arranged in such a way that the axial direction thereof runs in the front-to-rear direction (the vertical direction in Figure 1); a main upstream branch pipe 12 and downstream branch pipe 13 which are linked so as to extend coaxially with an angle of 180° between them on both sides of the outer peripheral surface of the chamber part 11; and a supplementary upstream branch pipe 14 which is formed above the chamber part 11.

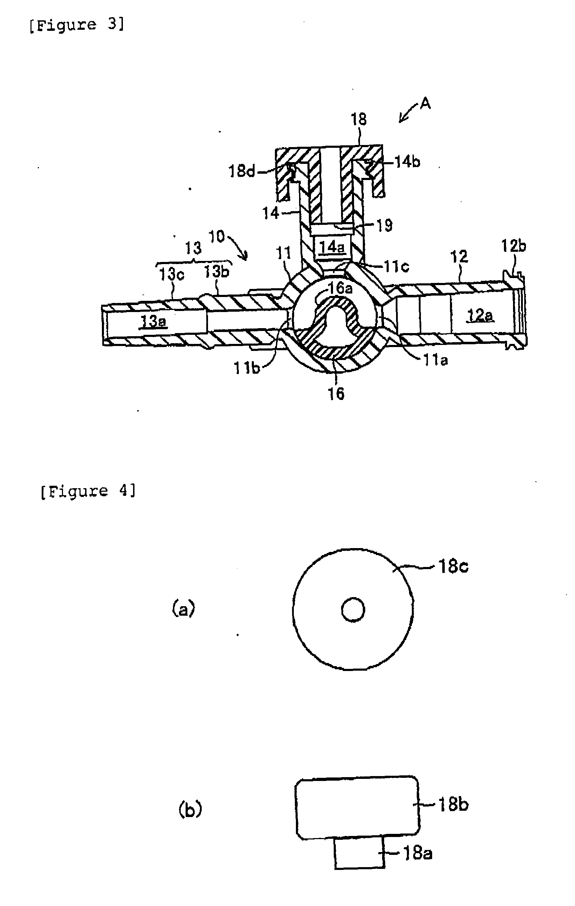

The chamber part 11 has a cylindrical shape with a bottom which is closed off at the rear end and open at the front end. As shown in Figure 3, three communicating holes 11a, 11b, 11c are then formed roughly in the centre in the axial direction of the chamber part 11. The communicating hole 11a is provided in correspondence with the main upstream branch pipe 12, and the inside of the chamber part 11 and a liquid flow channel 12a formed inside the main upstream branch pipe 12 are linked in communication by way of the communicating hole 11a. The main upstream branch pipe 12 has the shape of a female luer formed as a single piece with the chamber part 11. The liquid flow channel 12a formed inside the main upstream branch pipe 12 has an inner peripheral surface comprising a plurality of tapered portions having a large diameter on the upstream side (open side) and a small diameter on the downstream side (chamber part 11 side) and one straight portion.

That is to say, the downstream portion of the liquid flow channel 12a consists of a tapered portion comprising two levels with a smaller diameter at the communicating hole 11a side and a diameter which grows larger moving further away from the communicating hole 11a, and a straight portion formed on the upstream side thereof; the inclination of the downstream side is more pronounced than the inclination of the upstream side.

Furthermore, the upstream portion of the liquid flow channel 12a is formed with a gentle taper wherein the diameter becomes steadily larger approaching the opening of the main upstream branch pipe 12. A male thread 12b for linking is then formed on the outer periphery of the opening of the main upstream branch pipe 12. The communicating hole 11b is provided in correspondence with the downstream branch pipe 13, and the inside of the chamber part 11 and a liquid flow channel 13a formed inside the downstream branch pipe 13 are linked in communication by way of the communicating hole 11b.

The downstream branch pipe 13 is formed as a single piece with the chamber part 11, and it consists of a base end part 13b positioned on the chamber part 11 side, and a male luer part 13c positioned at the tip end side and formed to be narrower than the base end part 13b. The male luer part 13c has a tapered shape in which the tip end side becomes steadily narrower than the base end part 13b. Furthermore, the diameter of the upstream portion (chamber part 11 side) of the liquid flow channel 13a formed inside the downstream branch pipe 13 is formed to be substantially the same. The downstream portion (opening portion) of the liquid flow channel 13a has a smoothly tapering inner peripheral surface whereof the diameter on the upstream side is small, and the diameter on the downstream side becomes steadily greater.

Furthermore, the communicating hole 11c is provided in correspondence with the supplementary upstream branch pipe 14, and the inside of the chamber part 11 and a liquid flow channel 14a formed inside the supplementary upstream branch pipe 14 are linked in communication by way of the communicating hole 11c. The supplementary upstream branch pipe 14 has the shape of a female luer which is formed as a single piece with the chamber part 11, and the outer shape is substantially the same as the shape of the main upstream branch pipe 12. That is to say, the liquid flow channel 14a formed inside the supplementary upstream branch pipe 14 has a stepped (three-stage) tapering inner peripheral surface in which the diameter of the upstream side (opening side) is large, and the diameter of the downstream side (chamber part 11 side) is small.

The downstream portion of the liquid flow channel 14a is tapered, comprising two stages in which the diameter on the communicating hole 11c side is small and the diameter moving further away from the communicating hole 11c is large, and the inclination on the downstream side is greater than the inclination on the upstream side. Furthermore, the upstream portion of the liquid flow channel 14a tapers smoothly, with the diameter becoming steadily greater approaching the opening of the supplementary upstream branch pipe 14. A male thread 14b for joining the cap 18 (to be described later) is then formed on the outer periphery of the opening of the supplementary upstream branch pipe 14.

The rotary valve 15 consists of a valve body 16 and an operating part 17 which is linked to the front end of the valve body 16. The valve body 16 is arranged inside the chamber part 11, and can rotate in the axial direction inside the chamber part 11 by operation of the operating part 17. This valve body 16 has a substantially cylindrical outer shape, and a communicating groove part 16a running in the peripheral direction is formed on the peripheral surface of the central portion in the axial direction of the cylindrical shape. The communicating groove part 16a is formed so that the angle between the two lines linking both ends and the central part of the valve body 16 is somewhat greater than 180°, and, as shown in Figure 3, the communicating hole 11a and the communicating hole 11b are opposite the communicating groove part 16a when the central part in the axial direction of the communicating groove part 16a is oriented towards the communicating hole 11c. In this case, the liquid flow channels 12a, 13a, 14a are all in a communicating state via the communicating groove part 16a and the communicating holes 11a, 11b, 11c.

Furthermore, if the valve body 16 is turned clockwise from the state shown in Figure 3 so that the communicating hole 11b is facing the outer peripheral surface of the valve body 16, the liquid flow channel 13a is blocked from the other liquid flow channels 12a, 14a, and the liquid flow channels 12a, 14a are maintained in a state of communication with each other. If, on the other hand, the valve body 16 is turned anti-clockwise from the state shown in Figure 3 so that the communicating hole 11a is facing the outer peripheral surface of the valve body 16, the liquid flow channel 12a is blocked from the other liquid flow channels 13a, 14a, and the liquid flow channels 13a, 14a are maintained in a state of communication with each other. In addition, if the valve body 16 is turned through 180° in either direction so that the communicating hole 11c is facing the outer peripheral surface of the valve body 16, the liquid flow channel 14a is blocked from the other liquid flow channels 12a, 13a, and the liquid flow channels 12a, 13a are maintained in a state of communication with each other.

The operating part 17 comprises three operating pieces 17a, 17b, 17c which are formed with a certain angle between them, so that said operating pieces 17a, 17b, 17c correspond to the main upstream branch pipe 12, downstream branch pipe 13 and supplementary upstream branch pipe 14, respectively. That is to say, as shown in Figure 2, when the operating piece 17a is in a position pointing to the main upstream branch pipe 12, when the operating piece 17b is in a position pointing to the downstream branch pipe 13, and when the operating piece 17c is in a position pointing to the supplementary upstream branch pipe 14, the main upstream branch pipe 12, downstream branch pipe 13 and supplementary upstream branch pipe 14 are all in a state of communication via the chamber part 11. That is to say, the valve body 16 is in the state shown in Figure 3.

When the operating part 17 is turned through 90° in a clockwise direction from the state shown in Figure 2 so that the operating piece 17c is in a position pointing to the main upstream branch pipe 12 and the operating piece 17b is in a position pointing to the supplementary upstream branch pipe 14, the main upstream branch pipe 12 and the supplementary upstream branch pipe 14 are in communication, and the area between the supplementary upstream branch pipe 14 and the downstream branch pipe 13, and the area between the main upstream branch pipe 12 and the downstream branch pipe 13 are in a blocked state. Furthermore, when the operating part 17 is turned through 90° in an anti-clockwise direction from the state shown in Figure 2 so that the operating piece 17a is in a position pointing to the supplementary upstream branch pipe 14 and the operating piece 17c is in a position pointing to the downstream branch pipe 13, the supplementary upstream branch pipe 14 and the downstream branch pipe 13 are in communication, and the area between the main upstream branch pipe 12 and the supplementary upstream branch pipe 14, and the area between the main upstream branch pipe 12 and the downstream branch pipe 13 are in a blocked state.

In addition, when the operating part 17 is turned through 180° in either direction from the state shown in Figure 2 so that the operating piece 17a is in a position pointing to the downstream branch pipe 13 and the operating piece 17b is in a position pointing to the main upstream branch pipe 12, the main upstream branch pipe 12 and the downstream branch pipe 13 are in communication, and the area between the main upstream branch pipe 12 and the supplementary upstream branch pipe 14, and the area between the downstream branch pipe 13 and the supplementary upstream branch pipe 14 are in a blocked state. In this way, it is possible to ascertain from the position of the operating pieces 17a, 17b, 17c whether the areas between the main upstream branch pipe 12, downstream branch pipe 13 and supplementary upstream branch pipe 14 are in a communicating or blocked state.

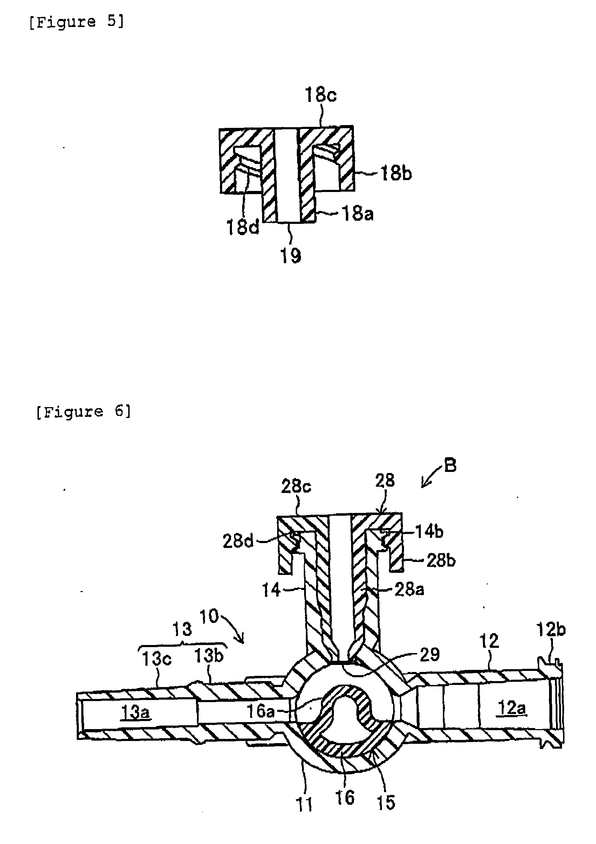

The cap 18 is fitted to the supplementary upstream branch pipe 14 to close off the liquid flow channel 14a of the supplementary upstream branch pipe 14 when the supplementary upstream branch pipe 14 is not being used, and it can be detached from the supplementary upstream branch pipe 14. As shown in Figures 4 and 5, the cap 18 consists of a cylindrical insertion part 18a (the cylindrical part according to the present invention) which can be inserted into the upstream portion of the supplementary upstream branch pipe 14, an outer peripheral wall 18b which can cover the upper part on the outer periphery of the supplementary upstream branch pipe 14, and an upper face part 18c which links the area between the upper ends of the insertion part 18a and outer peripheral wall 18b, and a female thread 18d which can screw together with the male thread 14b of the supplementary upstream branch pipe 14 is formed on the inner peripheral surface of the outer peripheral wall 18b.

The outer peripheral surface of the insertion part 18a has the shape of a tapering male luer in which the tip end has a somewhat narrower diameter than the base end, and it can come into tight contact with the inner peripheral surface of the upstream portion of the supplementary upstream branch pipe 14. Furthermore, the inner peripheral surface configures a gas expulsion channel which has substantially the same diameter from one end to the other. The outer peripheral wall 18b is designed to be held in the hand when the cap 18 is operated, and it is shaped like a cylinder which is short in the axial direction. The upper face part 18c is shaped like a circular plate provided with a hole in the centre which links in communication with the gas expulsion channel of the insertion part 18a.

A filter 19 is then provided at the tip end of the insertion part 18a, closing off the end part. This filter 19 consists of a hydrophobic filter made of nylon or cellulose, and its thickness is set at 0.25 mm. The filter 19 is configured so that gases such as air can pass through it, but liquids such as drug solutions and blood cannot pass through it. When air inside the supplementary upstream branch pipe 14 has passed through filter 19 in the state shown in Figure 3, this air is expelled to the outside by passing from the inside of the insertion part 18a through the hole in the upper face part 18c. Moreover, the filter 19 is formed by fusing or joining a sheet made of nylon or cellulose to the tip end of the insertion part 18a by applying pressure and applying very light ultrasonic waves.

With this configuration, when a certain drug solution is supplied to a patient's body (not depicted), the rear end of transfusion tube (not depicted) to which is connected an indwelling needle for puncturing the patient's body to become indwelling is connected to the downstream branch pipe 13. Furthermore, a male luer part provided at the tip end of the transfusion tube running from a container for housing the drug solution which is to be supplied to the patient is connected to the main upstream branch pipe 12. Then, if the supplementary upstream branch pipe 14 is not being used, the operating part 17 is operated in a state in which the cap 18 is fitted to the supplementary upstream branch pipe 14 to achieve the state shown in Figure 3, and the drug solution in the container is sent out, whereby the drug solution flows towards the downstream branch pipe 13 from the upstream branch pipe 12 side, passing through the chamber part 11. By means of this, air inside the liquid flow channels 12a, 13a and the communicating groove part 16a in the medical stopcock A with a cap is pushed out and expelled to the outside from the indwelling needle on the downstream side.

Next, when the rotary valve 15 is turned to a position in which the main upstream branch pipe 12, downstream branch pipe 13 and supplementary upstream branch pipe 14 are respectively linked in communication via the chamber part 11 so that the drug solution continues to flow from the main upstream branch pipe 12 side into the chamber part 11, the air inside the supplementary upstream branch pipe 14 is pushed upwards by the drug solution, passing through the filter 19 so that it is expelled to the outside from the gas expulsion channel in the cap 18. This means that when the space inside the medical stopcock A with a cap is completely filled with drug solution, the operating part 17 is operated and delivery of the drug solution is stopped for a time. Then, with the indwelling needle having punctured the patient's body and remaining indwelling, the operating part 17 is once again operated to send out the drug solution in the container towards the patient's body, whereby the drug solution is supplied to the patient. By means of this, it is possible to prevent air from entering the patient's body together with the drug solution, which also makes it possible to prevent air from accumulating inside the medical stopcock A with a cap.

Furthermore, when another drug solution is to be supplied to the patient in addition to the drug solution in the container, the cap 18 is removed from the supplementary upstream branch pipe 14, and a transfusion tube having a connector connected to the tip end is connected to the supplementary upstream branch pipe 14. The other drug solution is then delivered into the chamber part 11 from the supplementary upstream branch pipe 14. By means of this, two types of drug solution are supplied to the patient's body. In this case too, when the other drug solution is delivered into the chamber part 11 from the supplementary upstream branch pipe 14, an operation is carried out beforehand to remove the air inside the supplementary upstream branch pipe 14 and the air inside the transfusion tube, after which the drug solution is delivered.

In this way, with the medical stopcock A with a cap according to this mode of embodiment, when a drug solution flows from the main upstream branch pipe 12 towards the downstream branch pipe 13 and the supplementary upstream branch pipe 14 is not being used, the cap 18 to which the filter 19 is attached is fitted to the supplementary upstream branch pipe 14. The filter 19 allows gas to pass through, but does not allow liquid to pass through. Consequently, if the drug solution flows from the main upstream branch pipe 12 towards the downstream branch pipe 13, the air inside can be expelled to the outside from the hole in the puncture needle before the drug solution is supplied to the patient, and in addition, even if there is an air pocket inside the supplementary upstream branch pipe 14, that air is subjected to the pressure of the liquid, so that said air passes through the filter 19 and can be expelled to the outside.

By means of this, it is possible to prevent air from being delivered to the patient's body when the drug solution is supplied to the patient's body. It is also possible to prevent the accumulation of liquid which causes air pockets, and therefore it is possible to prevent bacterial growth inside the medical stopcock A with a cap. Furthermore, when the medical stopcock A with a cap is not being used, the cap 18 is fitted to the supplementary upstream branch pipe 14, which makes it possible to prevent bacteria and soiling from entering the medical stopcock A with a cap.

Furthermore, the filter 19 is formed like a thin film with a thickness of 0.25 mm, and it is fitted to the tip end opening of the insertion part 18a, and therefore it is simple to fit the filter 19 to the insertion part 18a, and also the filter 19 does not become readily detached from the insertion part 18a. In addition, a male thread 14b is provided on the outer peripheral surface of the supplementary upstream branch pipe 14, and also a female thread 18d which can screw together with the male thread 14b is provided on the inner peripheral surface of the outer peripheral wall 18b, and the cap 18 can be fitted to the supplementary upstream branch pipe 14 by screwing together the male thread 14b and the female thread 18d, and therefore the cap 18 can be simply attached to or detached from the supplementary upstream branch pipe 14.

### (Mode of Embodiment 2)

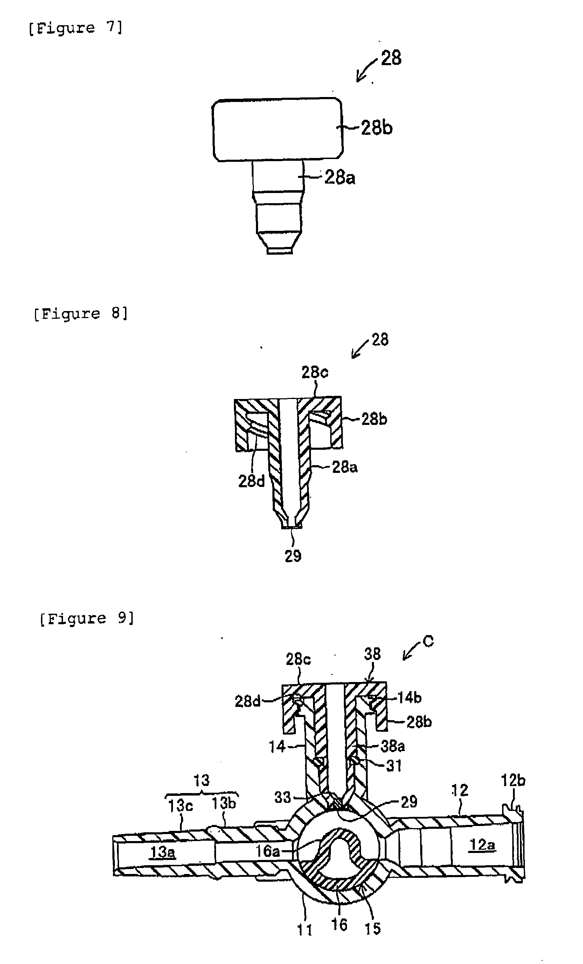

Figure 6 shows a medical stopcock B with a cap according to Mode of Embodiment 2 of the present invention. With this medical stopcock B with a cap, the parts other than the cap 28 and filter 29 have the same structures as those of the medical stopcock A with a cap described above. Similar parts therefore bear the same reference symbols, and they will not be described here. The cap 28 is configured in the manner shown in Figures 7 and 8. The cap 28 consists of a cylindrical insertion part 28a which can be inserted into the supplementary upstream branch pipe 14 in a state in which it is in contact with the whole of the inner peripheral surface of the supplementary upstream branch pipe 14, an outer peripheral wall 28b which can cover the upper part of the outer periphery of the supplementary upstream branch pipe 14, and an upper face part 28c which links the area between the upper ends of the insertion part 28a and outer peripheral wall 28b; a female thread 28d which can screw together with the male thread 14b is formed on the inner peripheral surface of the outer peripheral wall 28b.

The outer peripheral surface of the insertion part 28a has a stepped tapering shape in which the tip end is somewhat narrower than the base end, and it can come into tight contact with the inner peripheral surface of the supplementary upstream branch pipe 14. Furthermore, the outer peripheral wall 28b has the same shape as the outer peripheral wall 18b described above, the upper face part 28c has the same shape as the upper face part 18c described above, and the female thread 28d has the same shape as the female thread 18d described above. The filter 29 provided at the tip end of the insertion part 28a has the same configuration as the filter 19 described above, apart from the fact that it has a smaller diameter than the filter 19. Accordingly, when the cap 28 is fitted to the supplementary upstream branch pipe 14, the filter 29 lies inside the communicating hole 11c.

According to this medical stopcock B with a cap, when the supplementary upstream branch pipe 14 is not being used, the inside of the supplementary upstream branch pipe 14 does not constitute a liquid flow channel, and therefore there is no longer any space inside the supplementary upstream branch pipe 14 where air accumulates. In this case, the liquid flow channel 14a is no longer formed. The other operational effects of this medical stopcock B with a cap are the same as those of the medical stopcock A with a cap described above.

### (Mode of Embodiment 3)

Figure 9 shows a medical stopcock C with a cap according to Mode of Embodiment 3 of the present invention. With this medical stopcock C with a cap, a sealing member 31 is provided on the outer peripheral surface of an insertion part 38a of a cap 38, and also a one-way valve 33 is provided inside the insertion part 38a of the cap 38; parts other than this have the same structures as those of the medical stopcock B with a cap described above. Similar parts therefore bear the same reference symbols, and they will not be described here.

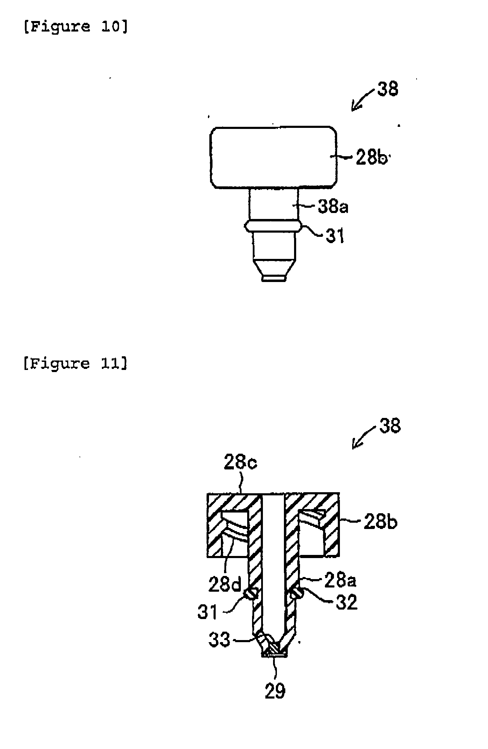

The cap 38 is configured in the manner shown in Figures 10 and 11. A groove part 32 is formed around the circumference at a fairly low portion of the outer peripheral surface of the insertion part 38a of the cap 38, and the sealing member 31 comprising a rubber ring is fitted inside said groove part 32. Furthermore, the one-way valve 33 consists of a rubber valve provided at the upper surface of the filter 29 inside the insertion part 38a, and it allows air which comes from the chamber part 11 and passes through the filter 29 to travel to the outside, but does not allow external air to pass through to the filter 29 side.

According to this medical stopcock C with a cap, when the cap 38 is fitted to the supplementary upstream branch pipe 14, it is possible to prevent air from entering the liquid flow channel of the medical stopcock C with a cap from the area between the insertion part 38a and the inner peripheral surface of the supplementary upstream branch pipe 14. Furthermore, it is possible to prevent air from entering the supplementary upstream branch pipe 14 when the cap 38 is removed from the supplementary upstream branch pipe 14 to connect a transfusion tube (not depicted) for supplying another drug solution to the supplementary upstream branch pipe 14. In this case, when the cap 38 is first of all withdrawn from the supplementary upstream branch pipe 14, the inside of the supplementary upstream branch pipe 14 is under negative pressure because of the sealing provided by the sealing member 31, and the liquid surface of the drug solution rises as the cap 38 is lifted up.

Next, if the rotary valve 15 is operated to block the area between the supplementary upstream branch pipe 14 and the chamber part 11, with the cap 38 having been withdrawn and the liquid surface having reached the opening of the supplementary upstream branch pipe 14, the liquid surface stays as it is. In this state, a transfusion tube can be connected to the supplementary upstream branch pipe 14 without air entering the supplementary upstream branch pipe 14, by connecting the transfusion tube which is filled with another drug solution to the supplementary upstream branch pipe 14. The other operational effects of this medical stopcock C with a cap are the same as those of the medical stopcock B with a cap described above.

Furthermore, the medical stopcock with a cap according to the present invention is not limited to the modes of embodiment described above, and various modifications can be made. For example, in the modes of embodiment described above, the valve body 16 of the rotary valve 15 has a configuration in which the communicating groove part 16a running in the peripheral direction is formed on the peripheral surface of a substantially cylindrical body, but the rotary valve is not limited to this configuration, and use may be made of a valve which is provided with a wall for allowing the liquid flowing from the main upstream branch pipe towards the downstream branch pipe to pass through the top of the inside of the chamber part for a time. It is also possible to use a rotary valve in which a liquid flow channel is provided inside the cylindrical body. In addition, the shape of the cap and the shape, material and thickness etc. of the filter may be appropriately modified.

### Brief Description of the Figures

[Figure 1] is a plan view of the medical stopcock with a cap according to Mode of Embodiment 1 of the present invention;
[Figure 2] is a front view of the medical stopcock with a cap according to Mode of Embodiment 1;
[Figure 3] is a view in cross section of the medical stopcock with a cap according to Mode of Embodiment 1;
[Figure 4] shows the cap with which the medical stopcock with a cap according to Mode of Embodiment 1 is provided, where (a) is a plan view, and (b) is a front view;
[Figure 5] is a view in cross section of the cap with which the medical stopcock with a cap according to Mode of Embodiment 1 is provided;
[Figure 6] is a view in cross section of the medical stopcock with a cap according to Mode of Embodiment 2 of the present invention;
[Figure 7] is a front view showing the cap with which the medical stopcock with a cap according to Mode of Embodiment 2 is provided;
[Figure 8] is a view in cross section showing the cap with which the medical stopcock with a cap according to Mode of Embodiment 2 is provided;
[Figure 9] is a view in cross section of the medical stopcock with a cap according to Mode of Embodiment 3 of the present invention;
[Figure 10] is a front view showing the cap with which the medical stopcock with a cap according to Mode of Embodiment 3 is provided; and
[Figure 11] is a view in cross section showing the cap with which the medical stopcock with a cap according to Mode of Embodiment 3 is provided.

### Key to Symbols

11...chamber part; 12...main upstream branch pipe; 13...downstream branch pipe; 14...supplementary upstream branch pipe; 14b...male thread; 15...rotary valve; 16a...communicating groove part; 18, 28, 38...cap; 18a, 28a, 38a...insertion part; 18b, 28b...outer peripheral wall; 18d, 28d...female thread; 19, 29...filter; 31...sealing member; A, B, C...medical stopcock.

## Claims

1. Medical stopcock with a cap, comprising:
a chamber part;
a main upstream branch pipe, downstream branch pipe and
supplementary upstream branch pipe which are respectively connected to the abovementioned chamber part;
a rotary valve provided inside the abovementioned chamber part which can form a liquid flow channel allowing communication between the abovementioned chamber part and the abovementioned main upstream branch pipe, the abovementioned downstream branch pipe and the abovementioned supplementary upstream branch pipe, respectively, and also which can block the area between the abovementioned chamber part and the abovementioned main upstream branch pipe, the area between the abovementioned chamber part and the abovementioned downstream branch pipe, and the area between the abovementioned chamber part and the abovementioned supplementary upstream branch pipe, respectively, by rotation of said valve in a certain direction; and
a cap which can be attached to or detached from the abovementioned supplementary upstream branch pipe,
said medical stopcock with a cap being **characterized in that** the abovementioned cap comprises a cylindrical part which can be inserted into the abovementioned supplementary upstream branch pipe, and a filter attached to the abovementioned cylindrical part allowing the passage of gas but not allowing the passage of liquid; and when the abovementioned cap is attached to the abovementioned supplementary upstream
branch pipe with the abovementioned cylindrical part
inserted into the abovementioned supplementary upstream
branch pipe, liquid can no longer move between the
abovementioned chamber part and the abovementioned
supplementary upstream branch pipe.

2. Medical stopcock with a cap according to Claim 1, in which the abovementioned filter consists of a thin-film hydrophobic filter.

3. Medical stopcock with a cap according to Claim 1 or 2, in which a male thread is provided on the outer peripheral surface of the abovementioned supplementary upstream branch pipe, an outer peripheral wall for covering the outer peripheral surface of the abovementioned supplementary upstream branch pipe is provided on the abovementioned cap, and also a female thread which can screw together with the abovementioned male thread is provided on the inner peripheral surface of the abovementioned outer peripheral wall.

4. Medical stopcock with a cap according to any one of Claims 1 to 3, in which the abovementioned filter is attached to the tip end opening of the abovementioned cylindrical part, and the abovementioned filter is positioned at the boundary area between the abovementioned chamber part and the abovementioned main upstream branch pipe.

5. Medical stopcock with a cap according to any one of Claims 1 to 4, in which a seal for sealing the area between the abovementioned cylindrical part and the inner peripheral surface of the abovementioned supplementary upstream branch pipe is provided around the circumference of the abovementioned cylindrical part.
